# EUROPÄISCHE PATENTSCHRIFT

(11) **EP 2 249 866 B1**
(45) Veröffentlichungstag und Bekanntmachung des Hinweises auf die Patenterteilung: **15.08.2012**
(21) Anmeldenummer: 09701974.9
(22) Anmeldetag: 14.01.2009
(51) Int. Cl.: A61K 45/06, A61K 9/12, A61P 31/16

(54) **VERWENDUNG EINES ACETYLSALICYLSÄURESALZES ZUR BEHANDLUNG VIRALER INFEKTIONEN**
USE OF AN ACETYLSALICYLIC ACID SALT FOR THE TREATMENT OF VIRAL INFECTIONS
UTILISATION D'UN SEL D'ACIDE ACÉTYLSALICYLIQUE POUR LE TRAITEMENT D'INFECTIONS VIRALES

(30) Priorität: 14.01.2008 DE 102008004386
(43) Veröffentlichungstag der Anmeldung: 17.11.2010
(73) Patentinhaber: Activaero GmbH, 35285 Gemünden (DE)
(72) Erfinder: LUDWIG, Stephan, 48149 Münster (DE); SCHEUCH, Gerhard, 35288 Wohratal (DE); PLANZ, Oliver, 72108 Rottenburg (DE)
(74) Vertreter: Vossius & Partner
(86) Internationale Anmeldenummer: PCT/DE2009/000033
(87) Internationale Veröffentlichungsnummer: WO 2009/089822

(56) Entgegenhaltungen:
- WO-A2-02/098339
- WO-A2-2006/074114

## Beschreibung

### Gebiet der Erfindung

Die Erfindung betrifft eine neue Zusammensetzung enthaltend ein Salz von o-Acetylsalicylsäure mit einer basischen Aminosäure zur Verwendung bei der Prophylaxe oder Behandlung viraler Infektionen des Menschen oder von Tieren.

### Stand der Technik und Hintergrund der Erfindung

Die Influenza gehört nach wie vor zu den großen Suchen der Menschheit mit pandemischem Potential. Es gibt nur wenige Medikamente gegen die auslösenden Pathogene, die Influenza A Viren, welche alle direkt gegen das Virus gerichtet sind. Problem hierbei ist, dass sich relativ schnell Resistenzen entwickeln können. Desweiteren besteht eine Gefahr, dass die sich unter Geflügel epidemisch ausbreitende Vogelgrippe, induziert durch Infektionen mit dem H5 Influenza A Virus, auch auf Menschen übergreifen kann. Insbesondere Personen, die in Kontakt mit infiziertem Geflügel kommen, sind insofern hochgradig gefährdet. Besonders zu beachten ist hier, dass zunehmend von Insensitivitäten der H5N1 Viren gegen die wenigen zugelassenen Medikamente wie Oseltamivir berichtet wird. Es besteht daher dringender Bedarf an neuen und breit wirksamen anti-Influenza Medikamenten sowohl zur Prophylaxe als auch zur Behandlung viraler Infektionen, wobei die Medikamente möglichst keine Resistenzen auslösen sollten.

Aus der Literaturstelle WO2004/060360 A1 ist es bekannt, dass Acetylsalicylsäure den Transkriptionsfaktor NF-kB in Wirtzellen hemmen kann und bei dieser Hemmung des NF-kB Signalwegs essentielle viralen Bestandteile im Zellkern verbleiben und nicht mehr in virale Partikel eingebaut werden können. Aus dieser Literaturstelle ist auch die aerogene Gabe von Acetylsalicylsäure zur Prophylaxe oder Behandlung viraler Infektionen bekannt.

Beispielsweise aus der Literaturstelle DE 102 02 019 A1 sind Salze der Acetylsalicylsäure mit basischen Aminosäuren bekannt, wobei damit erhaltene Zubereitungen ausschließlich zur oralen Gabe hergerichtet sind. Aus dieser Literaturstelle ist desweiteren die Verwendung solcher Salze zur Behandlung von Erkrankungen des rheumatischen Formenkreises, Arthritiden, Neuralgien, Myalgien, Migräne, ischämischen Herzkrankheiten, Schlaganfall, Angina pectoris, myocardialem Infarkt, Bypass-Operationen, PTCA, Stent-Implantationen, zur Stimulation des Immunsystems bei HIV-Patienten, zur Tumorprophylaxe, zur Verlangsamung des kognitiven Verfalls bei dementiellem Syndrom, zur Hemmung der Gallensteinbildung und/oder der Behandlung diabetischer Erkrankungen bekannt. Die insofern bekannten Salze sind zudem unter dem Handelsnamen Aspisol® bereits als Medikamente zur Behandlung von Asthma, Heuschnupfen, Nasenschleimhautschwellungen oder chronischen Atemwegsinfektionen eingesetzt, und zwar neben der oralen Gabe auch zur Injektion. Bei allen diesen Erkrankungen handelt es sich nicht um Erkrankungen, die in direktem Zusammenhang mit viralen Infektionen durch Influenzaviren stehen.

Eine Verwendung von reiner Acetylsalicylsäure als antiviralem Agenz, welches inhalativ als Aerosol in die Atemwege bzw. die Lunge verabreicht wird, hat sich im Tiermodell grundsätzlich sehr gut bewährt. Beim Menschen kann Inhalation reiner Acetylsalicylsäure jedoch starke Reizungen der Atemwege hervorrufen. Außerdem hat es sich in Einzelfällen gezeigt, dass eine Inhalation von Acetylsalicylsäure bei einigen sensitiven Patienten zu Asthmaanfällen führen kann. Jedenfalls bei Asthmanatienten oder Personen mit einem Asthmarisiko wäre eine aerogene Applikation von Acetylsalicylsäure als anti-Influenza Medikament daher kontraindiziert.

### Technisches Problem der Erfindung

Der Erfindung liegt daher das technische Problem zu Grunde eine Formulierung mit Acetylsalicylsäure zur verwendung beider behandlung viraler Infektionen anzugeben, welche besonders gut verträglich ist und insbesondere das Risiko der Induktion von Asthmaanfällen zuverlässig vermeidet.

Grundzüge der Erfindung und bevorzugte Ausführungsformen Zur Lösung dieses technischen Problems lehrt die Erfindung eine Zusammensetzung enthaltend eine physiologisch wirksame Dosis eines Salzes der o-Acetylsalicylsäure mit einer natürlichen oder nichtnatürlichen basischen Aminosäure zur verwendung zur Prophylaxe oder Behandlung viraler Infektionen des Menschen oder von Tieren, insbesondere von Säugetieren und Vögeln. Im Rahmen der Erfindung sind insbesondere Infektionen mit natürlicherweise vorkommenden Wild-Typ Viren, nicht jedoch Infektionen mit gentechnisch modifizierten Viren als virale Infektionen bezeichnet.

Unter den Vögeln kommen zur Prophylaxe oder Behandlung insbesondere Nutzvögel, wie Hühner, Gänse, Enten, Poularden, Puten, Truthähne, Wachteln oder Tauben, in Frage, aber auch Singvögel.

Durch die Verwendung eines solchen Salzes wird insbesondere bei aerogener Verabreichung eine Irritation von Gewebe, wie von Schleimhäuten des Atemweges, dadurch vermieden, dass der Wirkstoff in der dargereichten Formulierung nicht sauer ist. Dadurch werden insbesondere Asthmaanfälle bei mit Asthma erkrankten Patienten oder gefährdeten Personen zuverlässig vermieden und eine breite Anwendung sowohl als Therapeutikum als auch als Prophylaktikum steht auf Grund der Freiheit von Nebenwirkungsrisiken praktisch nichts entgegen, zumal die Formulierung sogar als Mittel gegen Asthma bereits eingesetzt wird. Zudem wird, wie im Rahmen der Erfindung gefunden wurde, durch die Derivatisierung der Acetylsalicylsäure die Hemmung der viralen Replikation praktisch nicht geschwächt, was nicht zu erwarten war; sie ist teilweise sogar überraschenderweise leicht erhöht.

gemäβ des Erfindung ist die basische Aminosäure ausgewählt aus der Gruppe bestehend aus "Lysin, Arginin, Ornithin, Diaminobuttersäure, und Mischungen solcher Aminosäuren", wobei es sich vorzugsweise um ein Monoacetylsalicylat, handelt. Eine Aminosäure ist eine alpha-Aminocarbonsäure, wobei am alpha-Atom ein Wasserstoff oder ein beliebiger Rest als Seitenkette gebunden sein kann. Eine basische Aminosäure enthält in der Seitenkette eine basische Gruppe oder mehrere basische Gruppen, insbesondere eine Amingruppe. Die basische Aminosäure kann insbesondere D-Lysin, L-Lysin oder eine Mischung aus D-Lysin und i-Lysin sein.

Die Zusammensetzug enthalt zusätzlich ein Salz der o-Acetylsalicylsäure mit mit Glycin Das Gewichtsverhältnis Lysin zu Glycin in der Zusammensetzung kann dabei im Bereich von 100 : 1 bis 1:1, insbesondere 100 : 1 bis 10 : 1 liegen. Bevorzugt ist eine Mischung von Salzen der Aminosäuren Lysin und Glycin. Besonders bevorzugt ist ein Salz oder eine Mischung von Salzen, wie in der unter dem Handelsnamen Aspisol® käuflichen Zusammensetzung, beispielsweise in wäßriger Lösung, enthalten.

Im Rahmen der Erfindung können auch pharmazeutische Zusammensetzungen eingesetzt werden, welche Prodrugs enthalten, die vom Organismus nach der Einnahme bzw. Darreichung in erfindungsgemäß eingesetzte Wirkstoffe natürlicherweise verstoffwechselt werden.

Die Erfindungsgemäße Zusammensetzung ist zur Prophylaxe oder Behandlung einer Vielzahl viraler Infektionen verwenbar. Besonders geeignet ist die Zusammensetzung zur Prophylaxe oder Behandlung von Infektionen mit Negativstrang RNA Viren, wie Influenza Viren, vorzugsweise Influenza A Viren, insbesondere Viren vom H5-oder H7-Typ.

Es wurde aber auch gefunden, dass die erfindungsgemäß eingesetzte Substanz die Überexpression virusinduzierter Zytokine suppremiert ("Zytokinsturm"), welche NF-κB abhängig reguliert sind. Mit der erfindungsgemäßen Substanz kann folglich generell die Pathogenität vieler Viren reduziert werden, deren pathogenes Potential u.a. mit einer überschießenden Zytokinexpression zusammenhänge. Daher ist die erfindungsgemäß eingesetzte Zusammensetzung auch zur Behandlung und Prophylaxe von viralen Infektionen mit Coronavirus (SARS), Respiratory Syncytial Virus (RSV), Filoviren, wie Marburg Virus oder Ebola Virus, Arenaviren, wie Lassa Virus, Argentinische, Bolivianische oder Venezuelanische hemor-hagische Fieber Viren, Hantaviren, Flaviviren, wie Dengue Virus oder Gelbfieber Virus, Crimean-Congo hemorrhagic fever Virus, Rift Valley Fieber. Virus, Parainfluenza Viren (Typ 1, 2 and 3), Rhinoviren, humane Metapneumoviren (hMPV) und Epstein Barr Virus geeignet.

Die galenische Herrichtung einer erfindungsgemäß eingesetzten pharmazeutischen Zusammensetzung kann in fachüblicher Weise und grundsätzlich zur beliebigen Darreichung, beispielsweise oral, zur Injektion oder aerogen zur Inhalation, erfolgen. Geeignete feste oder flüssige galenische Zubereitungsformen sind beispielsweise Granulate, Pulver, Dragees, Tabletten, (Mikro-) Kapseln, Suppositorien, Sirupe, Säfte, Suspensionen, Emulsionen, Tropfen oder Lösungen zur Injektion (i.v., i.p., i.m., s.c.) oder Vernebelung (Aerosole), transdermale Systeme, sowie Präparate mit protrahierter Wirkstoff-Freigabe, bei deren Herstellung übliche Hilfsmittel wie Trägerstoffe, Spreng-, Binde-, Überzugs-, Quellungs-, Gleit- oder Schmiermittel, Geschmacksstoffe, Süßungsmittel und Lösungsvermittler Verwendung finden. Als Hilfsstoffe sei Magnesiumcarbonat, Titandioxid, Lactose, Mannit und andere Saccharide, Talcum, Milcheiweiß, Gelatine, Stärke, Zellulose und ihre Derivate, tierische und pflanzliche Öle wie Lebertran, Sonnenblumen-, Erdnuss- oder Sesamöl, Polyethylenglycole und Lösungsmittel, wie etwa steriles Wasser und ein- oder mehrwertige Alkohole, beispielsweise Glycerin, oder Mischungen solcher Lösungsmittelgenannt. Eine erfindungsgemäß verwendete pharmazeutische Zusammensetzung ist dadurch herstellbar, dass mindestens ein erfindungsgemäß verwendetes Salz in definierter Dosis mit einem pharmazeutisch geeigneten und physiologisch verträglichen Träger und ggf. weiteren geeigneten Wirk-, Zusatz- oder Hilfsstoffen mit definierter und vorgegebener Dosis gemischt und zu der gewünschten Darreichungsform hergerichtet ist. Beispiele geeigneter Herrichtungen zur oralen Gabe sind beispielsweise der Literaturstelle DE 102 02 019 A1 und den darin genannten Literaturstellen entnehmbar.

Bevorzugt ist allerdings die galenische Herrichtung zur aerogenen, nasalen Darreichung als flüssige, wäßrige Zusammensetzung (Lösung), oder als Pulver, ggf. in Suspension in einem Treibmittel, beispielsweise einem bei Raumtemperatur verflüssigbaren Treibmittel, wie FCKW, Hydrofluoralkane, wie 1,1,1,2-Tetraflourethan oder 1,1,1,2,3,3,3-Heptafluorpropan, Propan, Butan, Isobutan, oder andere im Bereich der medizinischen Aerosolformulierungen üblichen Treibgase. Zusätzlich zu den genannten Treibmitteln oder ersatzweise kann auch Luft, Sauerstoff, Stickstoff, Kohlendioxid oder Lachgas eingesetzt werden. Dabei kann die Zusammensetzung die fachüblichen Zusatz- und Hilfsstoffe der medizinischen Aerosolformulierungen enthalten, wie beispielsweise physiologisch verträgliche oberflächenaktive Substanzen, und/oder fachübliche Suspendierhilfsmittel.

Die wäßrige Zusammensetzung ist weiterhin vorzugsweise eine 0,01 mM bis 3,0 M, vorzugsweise 0,5 bis 3,0 M, oder 0,01 bis 100 mM, insbesondere 0,1 bis 10 mM, wäßrige Lösung des Salzes oder der Mischung von Salzen.

Bevorzugt ist es, wenn die Partikelgröße des Aerosols, sei es die Lösung, sei es das Salz als Feststoff bzw. in Suspension, einen MMAD-Wert (Mass Median Aerodynamic Diameter) kleiner 10 µm, vorzugsweise kleiner 5 µm, aufweist. Zur Messung der aerodynamischen Partikelgrößenverteilung FPD (Fine Particle Dose) oder FPF (Fine Particle Fraction) eignen sich Impaktoren, wie zum Beispiel der 5-Stufen Multistage Liquid Impinger (MSLI) oder 8-Stufen Andersen Kaskaden Impaktor (ACI), die in Kapitel 601 der United States Pharmacopoeia (USP) oder in der Inhalanda Monographie der Europäischen Pharmakopöe (Ph. Eur.) beschrieben sind. Anhand der aerodynamischen Partikelverteilung kann mittels eines "Log-probability plots" der MMAD Wert einer Aerosol Zubereitung berechnet werden. Mit den bevorzugten MMAD Werten wird erreicht, dass die Aerosolteilchen lungengängig sind, und zwar bis in die tiefen Bereich der Lunge, so dass eine ausreichende Konzentration in der gesamten Lunge erreicht wird bei üblichen Dauern der Verabreichung. Um zu vermeiden, dass kleine Partikel wieder ausgeatmet werden, kann als Untergrenze für den MMAD Wert 0,1 µm, vorzugsweise 0,5 µm, höchstvorzugsweise 1 µm vorgesehen sein.

Das erfindungsgemäß eingesetzte Salz kann zur Erreichung der gewünschten MMAD Werte in fachüblicher Weise zerkleinert bzw. mikronisiert werden, beispielsweise mittels Stift-, Kugel-, oder Luftstrahlmühlen.

Zur Darreichung eines erfindungsgemäßen Aerosols kann mit allen im Bereich der Medizintechnik üblichen Inhalationsvorrichtungen mit Aerosolerzeugern, wie Zerstäubern bzw. Verneblern, erfolgen. Beispiele sind Pulver-Aerosol Zerstäuber bzw. DPI (Dry Powder Inhalatoren), Düsen-, Ultraschall-, oder Schwingmembranvernebler. Ebenfalls einsetzbar sind Aerosolerzeuger, die auf Basis des elektrohydrodynamischen Prinzips funktionieren oder Kondensationsaerosole aus flüssiger Formulierung. Beispiele für geeignete Inhalationsvorrichtungen sind in den Literaturstellen EP 1741460 A, EP 1700614 A, EP 1258264 A und EP 1163921 A beschrieben.

Die Konzentration des Salzes in der Zusammensetzung in Verbindung mit der Durchsatzrate eines eingesetzten Aerosolerzeugers ist mit der Maßgabe gewählt, dass zumindest 10 mg, vorzugsweise zumindest 50 mg, höchstvorzugsweise zumindest 100 mg, des Salzes innerhalb eines Zeitraumes von weniger als 5 min., vorzugsweise 2 min., höchstvorzugsweise 1 min., zu dem Aerosol umgewandelt und einem Patienten dargereicht werden.

Des Weiteren ist es für eine optimale Lungendeposition zweckmäßig, wenn der inspiratorische Fluss bzw. das inspiratorische Volumen kontrolliert und eingestellt wird. Denn bei einem zu schnellen Inhalationsfluss prallen die Aerosolteilchen bereits auf die Rachenhinterwand oder werden an der Stimmritze abgeschieden. Bei einer zu flachen Atmung gelangen die Aerosolteilchen nur in die oberen Atemwege und nicht in die tiefen Bereiche der Lunge. Ein eingesetztes Inhalationssystem sollte also eine tiefe, langsame Inhalation durch einen Patienten gewährleisten. Für Kinder sollte das Inhalationsvolumen zumindest 200 ml, vorzugsweise zumindest 500 ml, betragen. Für Erwachsene sollte das Inhalationsvolumen zumindest 300 ml, vorzugsweise zumindest 500 ml, betragen. Zweckmäßigerweise erfolgt eine Inhalation eines solchen Aerosolvolumens über eine Dauer von zumindest 1 s, besser zumindest 3 s, vorzugsweise zumindest 5 s. Der Inhalationsfluss wird vorteilhafterweise auf unter 1000 ml/s, insbesondere unter 500 ml/s, insbesondere unter 300 ml/s eingestellt, und das Atemvolumen auf mindestens 20%, insbesondere mindestens 30%, vorzugsweise mindestens 50%, bis zu 95%, bezogen auf die Inspirationskapazität eines Patienten. Letzteres gewährleistet eine einerseits langsame, andererseits aber auch tiefe Inhalation durch einen Patienten.

Somit betrifft die vorliegende Erfindung Zusammensetzungen zur Verwendung gemäß der Ansprüche 1 bis 9.

Im Rahmen der Erfindung können Inhalationssysteme eingesetzt werden, welche die vorstehenden Parameter meßtechnisch mittels Sensoren erfassen und mittels elektrischer, akustischer, und/oder optischer Signale über die ordnungsgemäße oder nicht ordnungsgemäße Inhalation informieren. Bezüglich dergestalt funktionierender Inhalationsgeräte wird ergänzend auf die vorstehenden Literaturstellen verwiesen. Im Falle einfacher Inhalationssysteme kann auch vorgesehen sein, dass eine Patienteninformationsschrift mit den genannten Maßgaben der pharmazeutischen Zusammensetzung beigefügt ist.

Daher betrifft die Erfindung auch eine Aerosolformulierung enthaltend eine Zusammensetzung gemäß Anspruch 1 bis 5, ein Treibmittel sowie optional Hilfs- und/oder Trägerstoffe. Das Salz kann in einer Menge von 0,001 bis 50 Gew.-%, 0,001 bis 10 Gew.-%, insbesondere 0,1 bis 10 Gew.-%, oder 10 bis 50 Gew.-%, insbesondere 30 bis 50 Gew.-%, bezogen auf die Gesamtformulierung, enthalten sein. Wenn das Salz in Partikelform vorliegt, kann es mit einem MMAD Wert von weniger als 10 µm, insbesondere weniger als 5 µm, enthalten sein.

Dabei ist insbesondere bei Einsatz der vorstehend erläuterten hohen Konzentrationen erstaunlich, dass Aerosolpartikel, die aus solchen Lösungen gebildet sind, mit der vorstehend angegebenen kleinen Partikelgröße herstellbar sind.

Ferner offenbart die Erfindung auch die Verwendung einer erfindungsgemäßen Aerosolformulierung zur Prophylaxe oder Behandlung viraler Infektionen des Menschen oder von Tieren, wobei einem Menschen, welcher an einer viralen Infektion zu erkranken droht oder hieran erkrankt ist, oder einem solchen Tier, eine physiologisch wirksame Menge der Aerosolformulierung aerogen zur Inhalation über die Nase oder den Mund dargereicht wird.

Schließlich offenbart die Erfindung eine Inhalationsvorrichtung mit einem Vorratsbehälter und einem an den Vorratsbehälter angeschlossenen Aerosolerzeuger, wobei der Vorratsbehälter eine erfindungsgemäße Aerosolformulierung enthält. An den Aerosolerzeuger ist ausgangsseitig üblicherweise ein Mundstück angeschlossen. Des Weiteren kann eine Luftpumpe vorgesehen sein, mittels welcher unter Steuerung durch einen Steuerungsvorrichtung der Inhalationsfluss und/oder das Inhalationsvolumen gesteuert werden. Es können Steuerungs- und/oder Regelungsmitteln zur Steuerung und/oder Regelung des Inhalationsflusses und des Atemvolumens vorgesehen sein, wobei die Steuerungs- und/oder Regelungsmittel vorzugsweise auf vorstehend genannte Werte eingestellt sind.

Die vorstehenden Erläuterungen zur erfindungsgemäßen pharmazeutischen Zusammensetzung treffen analog auch auf die erfindungsgemäße Aerosolformulierung, deren Verwendung, sowie auf die Inhalationsvorrichtung zu.

Im Folgenden wird die Erfindung anhand von Beispielen näher erläutert.

### Beispiel 1: Untersuchung von Resistenzphänomenen

Da besonders die Resistenzproblematik mit Acetylsalicylsäure als antiviral wirkendem Hemmstoff zellulärer Faktoren adressiert werden sollte, wurde die Tendenz zur Bildung resistenter Varianten vergleichend gegen die direkt auf das Virus gerichteten Medikamente Amantadin und Oseltamivir untersucht. A549 Lungenepithelzellen wurden mit dem hochpathogenen aviären Influenza A Virus Isolat A/FPV/Bratislava/79 (H7N7) (FPV, fowl plague virus, Geflügelpestvirus) mit einer MOI=0.01 (Multiplizität der Infektion) infiziert und für weitere 24h in An- oder Abwesenheit von Acetylsalicylsäure (5 mM), Amantadin (5 µM) und Oseltamivir (2 µM) inkubiert. In einem separaten Ansatz wurden A549 Lungenepithelzellen mit A/FPV/Bratislava/79 (H7N7) mit einer MOI=0.001 infiziert und für weitere 24h in An- oder Abwesenheit von Lysin-Glycin-Acetylsalicylat (5 mM) und Oseltamivir (2 µM) inkubiert. Bei beiden Ansätzen wurde dann der Zellüberstand jeder Probe wurde gesammelt und hieraus der virale Titer im Plaque-Assay auf MDCK Zellen bestimmt. Die Überstände wurden darauf hin normalisiert und wiederum genutzt, um mit jeweils gleicher Zahl an Viren eine zweite behandelte bzw. unbehandelte Zellpassage unter gleichen Bedingungen zu infizieren. Diese Prozedur wurde insgesamt bis zur fünften bzw. achten Passage wiederholt.

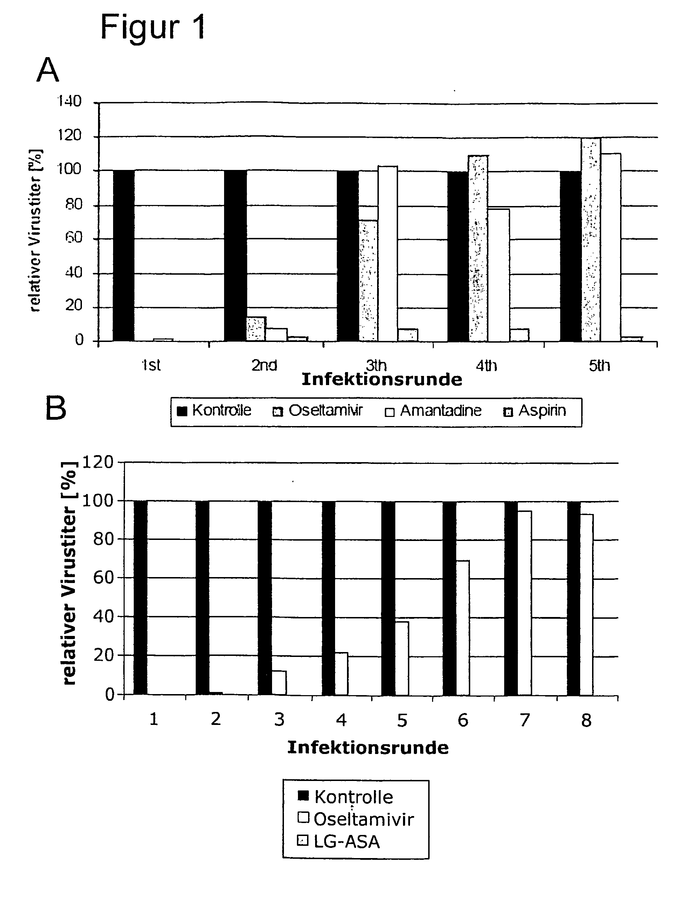

In Figur 1A wurden die Virustiter aus mit Acetylsalicylsäure bzw. Amantadin oder Oseltamivir-behandelten Zellen vergleichend zu den Titern aus den Überständen unbehandelter Zellen graphisch aufgetragen. Es zeigt sich bereits in der dritten Passage, dass die Virustiter aus Amantadin-behandelten Zellen aufgrund der Bildung resistenter Varianten wieder signifikant ansteigen. Überaschenderweise findet man dies im vergleichbaren Ausmaß unter den hier gewählten experimentellen Bedingungen auch für Oseltamivir. Im klaren Gegensatz hierzu findet man, dass Acetylsalicylsäure auch in der fünften Passage noch die unverändert gleiche antivirale Aktivität wie in der ersten Passage aufweist.
In Figur 1B wurden die Virustiter aus mit Lysin-Glycin-Acetylsalicylat bzw. Oseltamivir-behandelten Zellen vergleichend zu den Titern aus den Überständen unbehandelter Zellen graphisch aufgetragen. Es zeigt sich auch für Lysin-Glycin-Acetylsalicylat das in Figur 1A beschriebene Ergebnis, wobei selbst nach acht Passagen noch keine virale Resistenzbildung nach Lysin-Glycin-Acetylsalicylat Behandlung nachweisbar ist.
Die erhalten Ergebnisse sind in vollem Umfang geeignet, um die Aussage zu treffen, dass sowohl Acetylsalicylsäure als auch Lysin-Glycin-Acetylsalicylat keinerlei Tendenz zur Bildung resistenter Varianten in Zellkultur aufweiset.

### Beispiel 2: Untersuchung der Formulierung Lysin-Glycin-Acetylsalicylat auf antivirale Aktivitität gegen hochpathogene Influenza A Viren

Bei der untersuchten Zusammensetzung handelte es sich um Lys-Gly-Acetylsalicylat. (folgend auch LG-Acetylsalicylat genannt), welches der molekularen Zusammensetzung nach dem Produkt Aspisol®, erhältlich von der Bayer AG, entspricht.

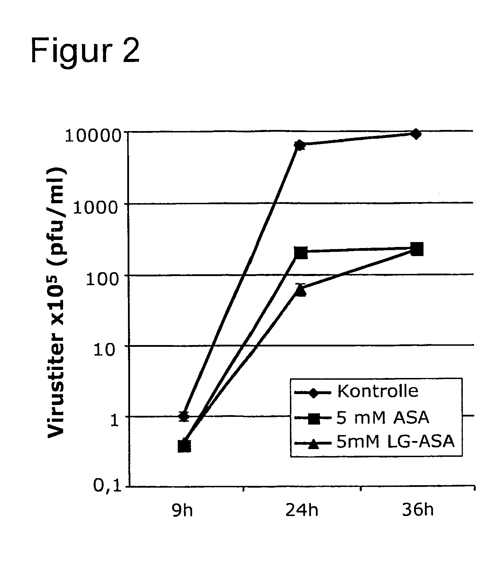

Diese Formulierung wurde in die Untersuchungsreihe auf antivirale Aktivität gegen Influenza Viren aufgenommen. A549 Lungenepithelzellen wurden mit A/FPV/Bratislava/79 (H7N7) (MOI=0.01) infiziert und für 8 h, 24 h und 36 h in An- oder Abwesenheit von Acetylsalicylsäure (5 mM), bzw. LG-Acetylsalicylat (5 mM) inkubiert. Der Zellüberstand jeder Probe wurde dann gesammelt und hieraus der virale Titer im Plaque-Assay auf MDCK Zellen bestimmt. Figur 2 zeigt eine graphische Auftragung der Virustiter über die Zeit. Im Ergebnis zeigt sich, dass LG-Acetylsalicylat ebenso effizient die Virusvermehrung des hochpathogenen aviären H7N7 Isolats hemmt wie Acetylsalicylsäure.

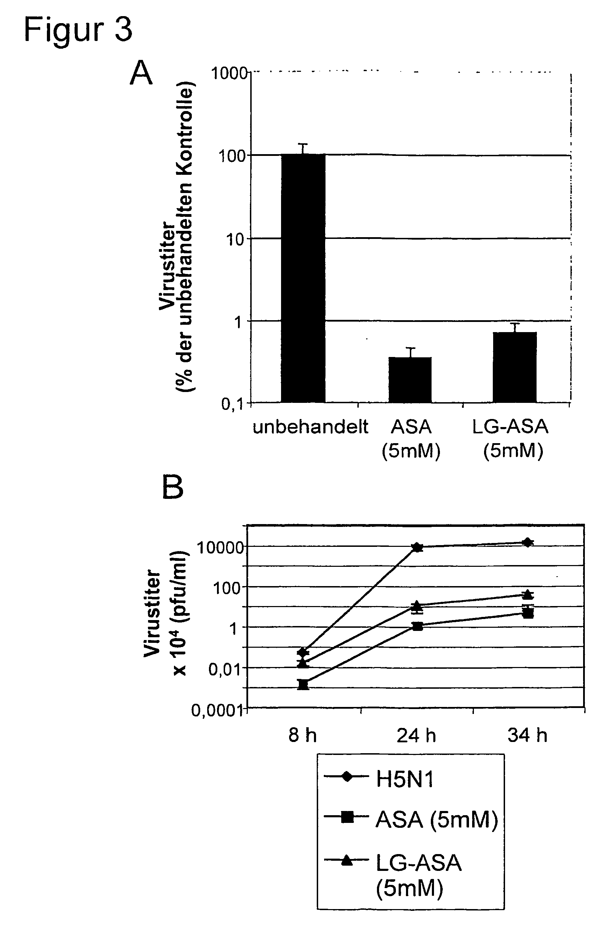

Dies ist, wie in Figur 3 gezeigt, auch der Fall nach Infektion mit hochpathogenen Isolaten des Subtyps H5N1. A549 Lungenepithelzellen wurden mit dem humanen H5N1 Isolat A/Thailand/KAN-1/2004 (MOI=0.001) infiziert und in An- oder Abwesenheit von Acetylsalicylsäure oder LG-Acetylsalicylsäure in den angegebenen Konzentrationen inkubiert. Der Zellüberstand wurde zur Bestimmung der Virustiter im Plaque-Assay auf MDCK Zellen untersucht. Figur 3A zeigt einen Zeitwert von 20 h, in Figur 3B sind die Virustiter in einer Wachstumskinetik aufgetragen. In beiden Fällen sieht man eine effiziente Hemmung der Virustiter des H5N1 Stammes um mehrere Zehnerpotenzen.

Aufgrund des hohen antiviralen Wirkpotentials in vitro ist davon auszugehen, dass sich die Formulierung LG-Acetylsalicylat als anti-Influenza Wirkstoff für eine inhalative Darreichung eignet.

### Beispiel 3: Einfluss von LG-Acetylsalicylat auf hochpathogene aviäre Influenzaviren im Zellkulturssystem

MDCKII Zellen wurden im MEM-Medium (MEM; Gibco (Invitrogen) Germany 21430-079, Lot 32034) mit Zusatz von 10% hitzedeaktivierten fötalem Kälberserum (FCS, PAA Laboratories /A04305-0346), Penicillin (Grünenthal/616G03) und Streptomycin (Sanavita/03056440111) kultiviert. Für die Infektion wurden die Zellen in 24 well-Platten ausgesät (8 x 10⁴ Zellen/well; Greiner, Germany, No. 662160, Lot 05210151) und über Nacht bei 37 °C inkubiert. Vor der Infektion wurden die Zellen mit PBS gewaschen, das entsprechende Virus (FPV, SN1, MB1) wurde in PBS/BA (PBS supplemented with 0,6% BA (MP Biomedicals), 1 mM MgCl₂, 0,9 mM CaCl₂, Penicillin (Grünenthal/616G03) und Streptomycin verdünnt und mit einer MOI von 0,001 auf den Zellrasen pipettiert. Nach einer Inkubationszeit von 30 min bei 37 °C wurde das Virusinokulum entfernt, zu den Zellen wurde entweder 1ml MEM-Medium oder MEM-Medium, welches 5mM LG-Acetylsalicylat enthielt, zugegeben. Nach 8, 24, 32 und 48 Stunden nach der Infektion wurde der jeweilige Überstand entnommen. Das Vorhandensein infektiöser Viruspartikel wurde in einem "plaque assay" nachgewiesen.

Für den "plaque assay" wurden MDCKII Zellen in 96 well Platten so ausgesät, dass der Zellrasen am nächsten Tag konfluent war. Die Zellen wurden mit PBS gewaschen und mit Verdünnungen der Überstände, welche in PBS/BA angelegt wurden, für 60 min bei 37°C infiziert. Nach der Inkubation wurden die Zellen mit einem Avicel-Medium-Gemisch (Avicel RC-581(FMC/B624C)) überschichtet. Hierzu wurde eine 2,5% Avicellösung mit der gleichen Menge 2x MEM-Medium gemischt. Nach einer Inkubationzeit von 20 h wurde das Avicel-Medium-Gemisch entfernt, die Zellen wurden mit einer 4% Roti®-Histofix (Roth/32789170) Lösung in PBS für 30 min bei 4°C fixiert und danach mit PBS gewaschen. Die für die Färbung notwendigen, folgenden Arbeitsschritte wurden bei Raumtemperatur durchgeführt. Durch die Inkubation mit 0,3% Triton-X-100 (Serva/30043) in PBS wurden die Zellen permeabilisiert. Virusinfizierte Zellen wurden durch ein immunhistologisches Verfahren gefärbt. Hierzu wurden die Zellen 1h mit einem monoklonalen Antikörper (Serotec/250107) inkubiert, der für das Influenza A Virus Nukleoprotein spezifisch ist. Die Detektion der infizierten Zellen erfolgte durch eine weitere Inkubation (30 min) mit einem Peroxidasegekoppelten anti-Maus Antikörper (DIANOVA/75790) und der Zugabe des True Blue™ Peroxidase Substrats (KPL/070490). Die Antikörperverdünnungen wurden in PBS mit 10% FCS und 0,1% Tween-20 (Serva/16211) angelegt. Nach der Inkubation mit dem primären und sekundärem Antikörper wurden die Zellen dreimal für 5 Minuten mit PBS/0,1% Tween-20 gewaschen. Zum Stoppen der Reaktion wurden die Platten mit Leitungswasser gewaschen und getrocknet. Getrocknete Platten wurden eingescannt und mit Hilfe der Corel DRAW 9.0 Software ausgewertet. Um den Virustiter der Überstände zu bestimmen wurden die Anhäufungen infizierter Zellen (Foci) in jedem well der 96 well Platte ausgezählt. Die Anzahl der ausgezählten Foci wurde mit dem jeweiligen Verdünnungsfaktor multipliziert. Aus den errechneten Werten wurde für jede Probe der Mittelwert bestimmt. Der Virustiter wurde als Log10 der Mittelwerte angegeben.

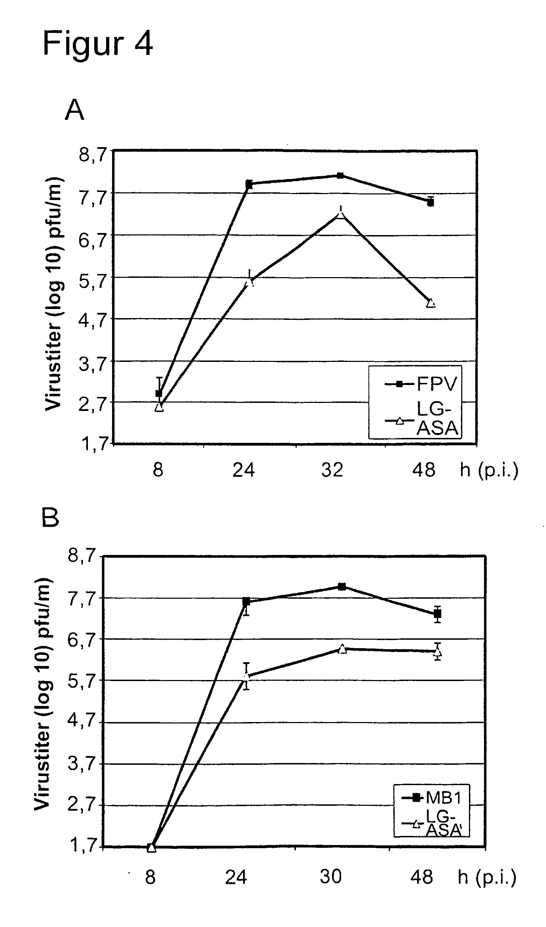

Die Ergebnisse sind in der Figur 4 dargestellt. Man erkennt, dass sowohl die Replikation eines H7N1 Virus (FPV) als auch eines H5N1 Virus (MB1) durch Behandlung mit LG-Acetylsalicylat im Zellkultursystem teilweise um mehr als 99% reduziert wird.

### Beispiel 4: Untersuchung der molekularen Wirkmechanismen, die der antiviralen Aktivität von LG-Acetylsalicylat zugrunde liegen.

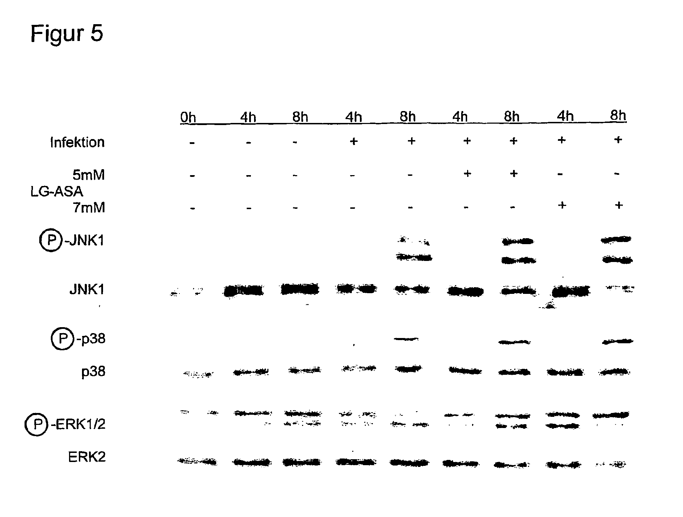

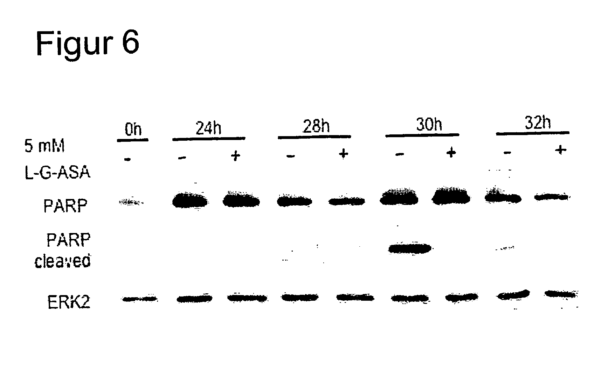

Im Weiteren galt es zu untersuchen, ob das molekulare Wirkprofil von LG-Acetylsalicylat vergleichbar mit dem der Reinsubstanz Acetylsalicylsäure ist. LG-Acetylsalicylat sollte als NF-kB Inhibitor wirken und entsprechend keine Nebeneffekte auf andere virus-induizerte Signalwege haben. Eine wichtige Gruppe an Signalmediatoren, welche auch nach Influenza Virus Infektion aktiviert werden, sind die sogenannten mitogen-aktivierten Protein Kinasen (MAPK). Hierzu gehören die Kinasen JNK, p38 und ERK. Für die Reinsubstanz Acetylsalicylsäure wurde bereits gezeigt, dass die virusinduzierte Aktivierung dieser Kinasen nicht durch Acetylsalicylsäure gehemmt wird. Wie Figur 5 zeigt, ist dies auch für LG-Acetylsalicylat der Fall: die virusinduzierte Aktivierung von JNK, p38 und ERK (Figur 5, Spur 5), die mit Hilfe phosphospezifischer Antikörper gegen die aktive Form dieser Kinasen im Western Blot nachgewiesen werden kann, ist durch Zugabe von 5mM (Spur 7) oder 7 mM Acetylsalicylsäure (Spur 9) nicht blockiert. Acetylsalicylsäure wirkt antiviral über die Hemmung der Expression proapoptotischer Faktoren, was schließlich zu einer verminderten Caspaseaktivierung in der Zelle führt. Figur 6 zeigt dies auch für LG-Acetylsalicylat anhand eines Western Blots, der Caspaseaktivierung über die Spaltung des Caspase Substrates Poly-ADP-Ribose-Polymerase (PARP) zeigt. Die nach 30 h deutlich sichtbare Bande des gespaltenen PARP (Spur 6) ist effizient reduziert in LG-Acetylsalicylat behandelten Proben (Spur 7).

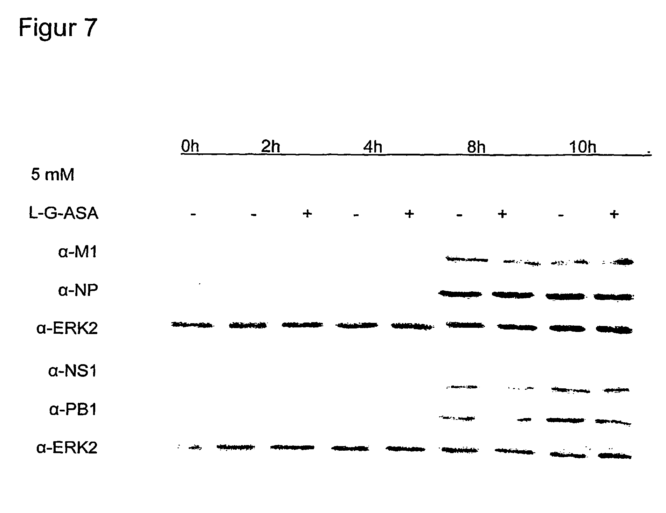

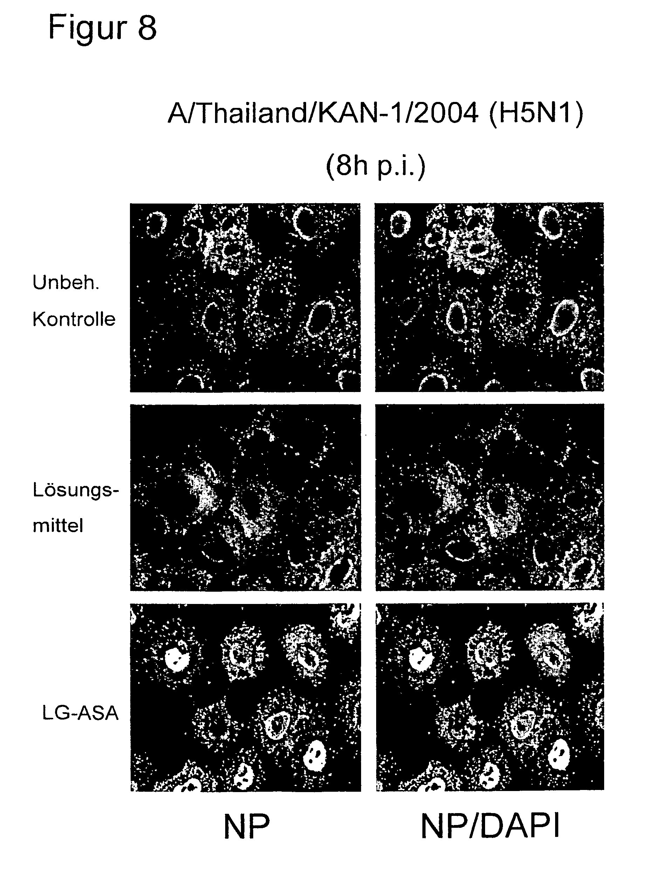

Der NF-kB abhängige Schritt in der Virusreplikation ist der Caspase-abhängige Export viraler Ribonukleoproteinkomplexe (RNP) ins Zytoplasma, welche dann an der Zellmembran in neue Viruspartikel eingebaut werden können. Acetylsalicylsäure blockiert diesen Schritt spezifisch, ohne auf die Akkumulation viraler Proteine in einer früheren Phase des Vermehrungszyklus zu wirken. Für LG-Acetylsalicylat gilt der identische Wirkmechanismus: Figur 7 zeigt in einem Western Blot, dass die Akkumulation der viralen Proteine M1, NP, NS1 und PB1 nicht durch LG-Acetylsalicylat gehemmt wird. Jedoch findet man, wie zuvor für die Reinsubstanz Acetylsalicylsäure beschrieben, ein effizentes Zurückhalten viraler RNP Komplexe, wie aus den in Figur 8 gezeigten Immunfloureszenzanalysen deutlich wird.

Damit lässt sich der Schluss ziehen, dass LG-Acetylsalicylsäure das gleiche antivirale Potential wie Acetylsalicylsäure hat und über die identischen molekularen Mechanismen hemmend auf die Virusvermehrung wirkt.

### Beispiel 5: Reduktion des IP10 und Interferon-gamma m-RNA Expressionslevel nach LG-Acetylsalicylat Behandlung

Es ist bekannt, dass eine überschiessende Zytokinproduktion, der sogenannte Zytokinsturm, ein wichtiger Pathogenitätsfaktor bei Infektionen durch H5N1 Influenza Viren ist. Da die meisten dieser Zytokine NF-κB abhängig reguliert sind, war zu prüfen, ob LG-ASA die Expression virusinduzierter Zytokine hemmen kann und somit zusätzlich indirekt die Pathogenität dieser Viren beeinflussen kann. Aus eigenen Vorarbeiten war bekannt, dass IP10 und IFN-gamma nach Infektion mit H5N1 (MB1) in Mauslungen hochreguliert wird. Die Expression dieses Chemokines bzw. Zytokins wird durch den Transkriptionsfaktor NF-κB induziert. Basierend auf früheren Erkenntnissen konnte gezeigt werden, dass Aspirin als NF-κB Inhibitor wirkt.

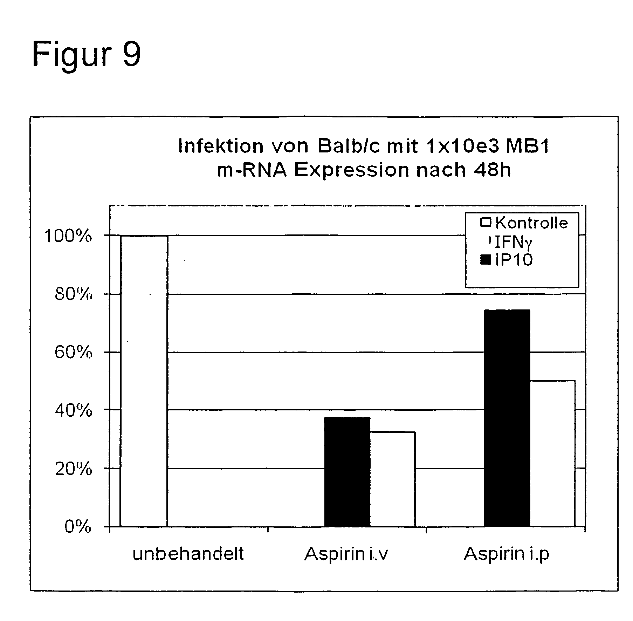

Ziel dieses Experimentes war es herauszufinden, ob LG-Acetylsalicylat als NF-κB Inhibitor in der Mauslunge nach H5N1 Infektion wirkt. Hierzu wurde untersucht, ob eine Behandlung der Mäuse vor und während der MB1 Infektion mit LG-Acetylsalicylat eine Auswirkung auf die Expressionsrate der beiden Chemokine bzw. Zytokine hat. Für das Experiment wurden je fünf Balb/c Mäuse eine Stunde vor Infektion (Infektionsdosis: 1x10³ pfu/50 µl) i.v. (100 µl) und i.p. (200 µl) mit 50mM LG-Acetylsalicylat behandelt. Die weiteren Behandlungen erfolgten 17, 24 und 42 Stunden nach Infektion. Nach 48 Stunden p.i. wurden die Lungen entnommen und die RNA isoliert. Die Expression von IP10 und IFN-gamma in den Mauslungen LG-Acetylsalicylat behandelter Mäuse im Vergleich zu den Kontrolltieren wurde Mittels quantitativer RT-PCR nachgewiesen. Die Daten sind in Figur 9 dargestellt.

Die Expression von IP10 und IFN-gamma bei unbehandelten Mäusen wurde gleich 100% gesetzt um somit eine klarere Vergleichsmöglichkeit zu schaffen. Nach viermaliger Behandlung mit LG-Acetylsalicylat war sowohl über die intravenösen als auch über die intraperitoneale Behandlungsroute eine Reduktion der m-RNA Expression bei beiden Chemokinen bzw. Zytokinen zu erkennen. Die intravenöse Behandlungsroute zeigte mit einer 62% Reduktion für IP10 und einer 68% für IFN-gamma einen stärkeren Effekt als bei der intraperitonealen Behandlungsroute mit 26% für IP10 und 50% für IFN-gamma.

Die Ergebnisse zeigen, dass die H5N1 Virus induzierte Expression NF-kB abhängiger Gene in LG-Acetylsalicylat behandelten infizierten Mauslungen stark reduziert ist. Dies ist insofern bedeutsam, als das die nach H5N1 Virus Infektion beobachtete überschiessende Produktion von Zytokinen ("Zytokinsturm"), welche größtenteils NF-kB abhängig reguliert werden, stark zur Pathogenität dieser Viren beiträgt. Somit kann LG-ASA nicht nur direkt die Virusreplikation beeinflussen, sondern auch indirekt den Krankheitsverlauf durch Reduktion überschiessender Zytokinproduktion positiv beeinflussen.

### Beispiel 6: Verträglichkeitsstudien

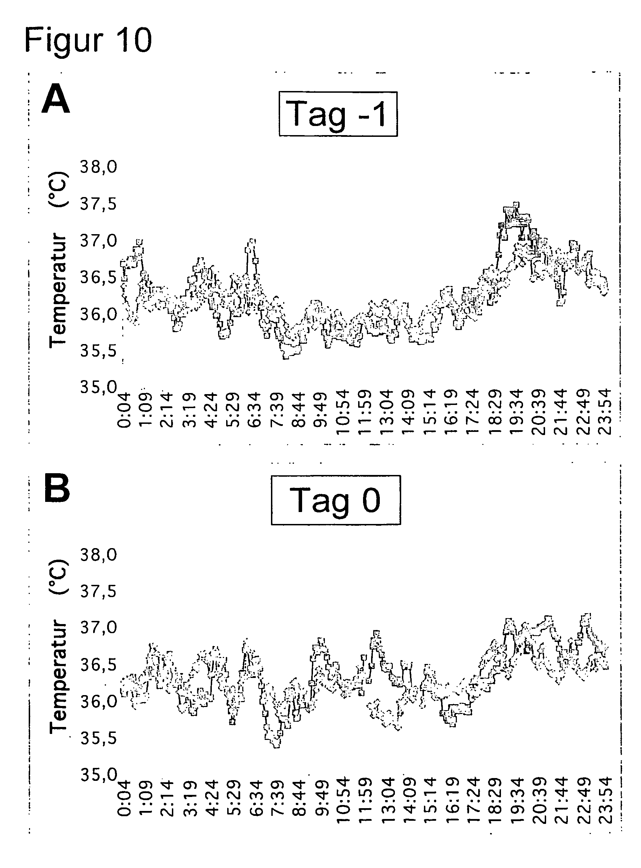

Im folgenden Experiment sollte die Verträglichkeit von LG-Acetylsalicylat nach Aerosolbehandlung von Mäusen getestet werden. Um den Einfluss der Behandlung bestmöglich zu beobachten, wurde das Experiment mit Hilfe des Maus Monitoring Systems durchgeführt. Dies erlaubt die Messung der Temperatur in Echtzeit. Alle 5 Minuten wurde ein Temperaturwert ermittelt und graphisch dargestellt (Figur10A-F). Weiterhin wurden die Tiere jeden Tag gewogen. Am Ende der Behandlung wurden die Mäuse getötet und es wurde das Organgewicht von Leber und Milz bestimmt. Bereits bei ersten Anzeigen von Lebertoxizität kommt es zur Vergrößerung der Leber. Eine Vergrößerung der Milz hingegen ist ein Zeichen für Entzündungsprozesse.

Für die Verträglichkeitsstudie wurden insgesamt 12 weiblichen Balb/c Mäuse 3 Tage vor Behandlungsbeginn ein Maus Monitoring Sender implantiert. Der erfolgreiche Eingriff wurde über 3 Tage beobachtet. Danach wurden 6 Mäuse dreimal täglich mit jeweils 2 ml einer 50 mM LG-Acetylsalicylat Lösung mit Hilfe eines Pari-Verneblers behandelt. Als Kontrollen dienten 6 Mäuse, die mit 2 ml PBS behandelt wurden. Die Lösungen wurden mit einem Druck von 1,5 bar benebelt, so dass die Behandlung ca. 10 Minuten dauerte. Die Behandlungen wurden täglich für 5 Tage um 9:00; 12:00 und 15:00 Uhr durchgeführt.

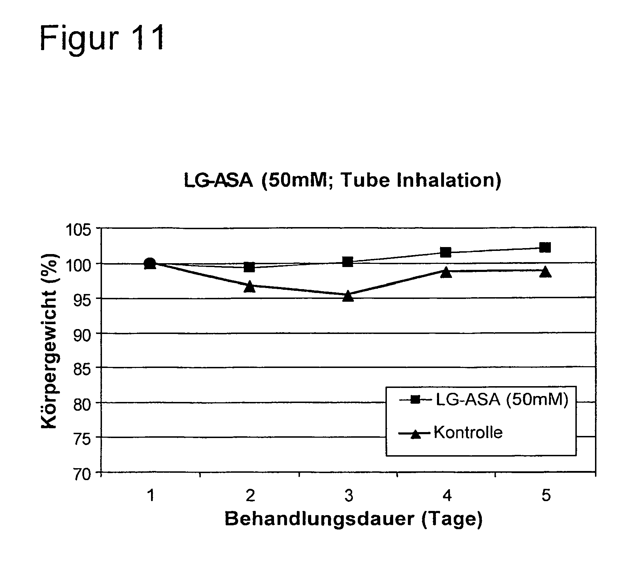

Untersucht wurde der Einfluss von LG-Acetylsalicylat auf den Gewichtsverlauf. Alle Mäuse wurden beginnend mit dem Behandlungstag täglich gewogen und zwar vor der ersten Behandlung (9:00 Uhr). Das Gewicht vom ersten Behandlungstag wurde als 100% gesetzt und das ermittelte Körpergewicht an den darauf folgenden Tagen prozentual in Relation gesetzt. Im Vergleich der beiden Behandlungsgruppen ergab sich die in Figur 11 dargestellte Graphik. Aus dieser geht hervor, dass es zwischen der LG-Acetylsalicylat Gruppe und der PBS behandelte Gruppe keinen signifikanten Unterschied im Bezug auf Veränderungen des Körpergewichtes gab.

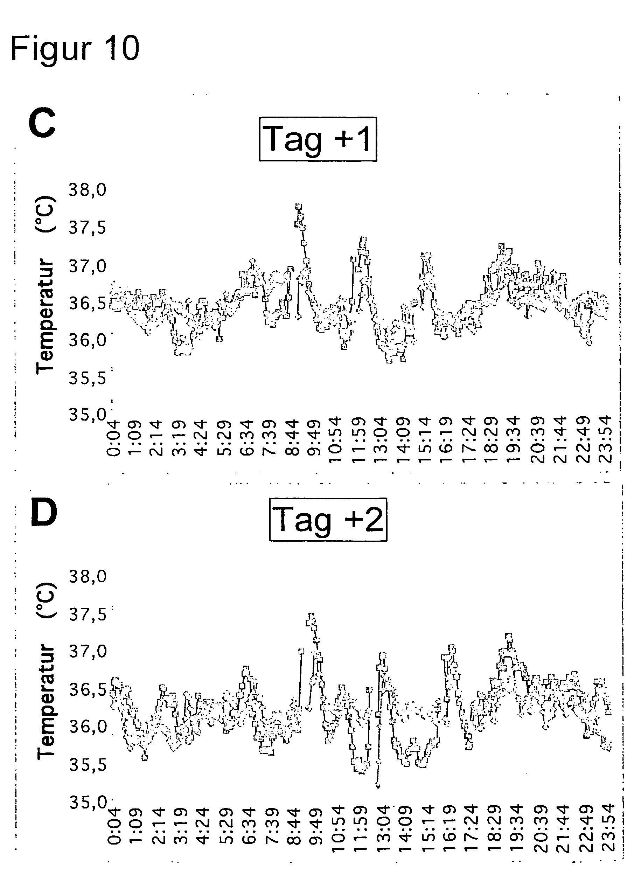

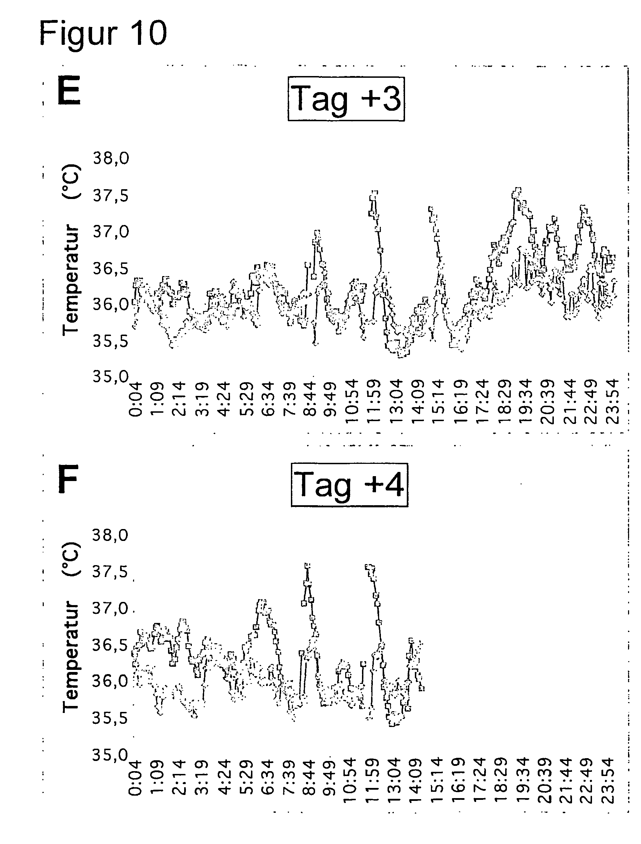

Weiterhin wurde der Einfluss von LG-Acetylsalicylat auf die Körpertemperatur untersucht. Wie bereits erwähnt, ermöglicht das Maus Monitoring System die Ermittlung der Körpertemperatur von Mäusen in Echtzeit. Da Mäuse einen hohen Stoffwechsel haben, führen bereits kleinste Veränderungen des Wohlbefindens zur Veränderung der Körpertemperatur. In Figur 10 ist der Verlauf der Körpertemperatur beginnend 1 Tag vor der Behandlung (Figur 10A) bis zum Ende der Behandlung (Tag +4) (Figur 10F) dargestellt. Es ist kein Unterschied bezüglich der Körpertemperatur bei LG-Acetylsalicylat behandelten Mäusen im Vergleich zu den Kontrolltieren zu erkennen.

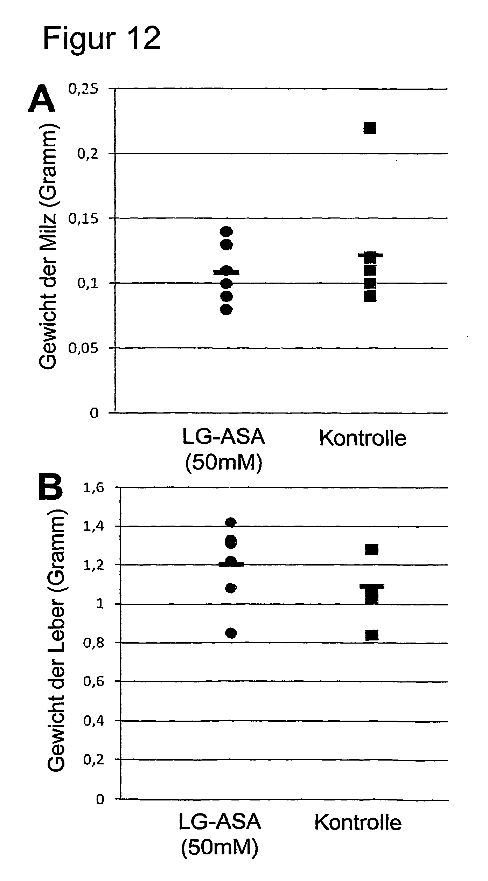

Schließlich wurde der Einfluss von LG-Acetylsalicylat auf das Gewicht von Leber und Milz untersucht. Fünfzehn Minuten nach der letzten Behandlung wurden alle Tiere getötet und es wurde eine Nekropsie durchgeführt. Die inneren Organe wurden nach kompletter Entblutung durch die Vena Cava begutachtet. Die Lungen wurden zuerst durch das Diaphragma in nicht zusammenfallendem Zustand begutachtet. Leber und Milz wurden gewogen. Die Ergebnisse sind in Figur 13 dargestellt. Da das Körpergewicht der Mäuse sich während der Behandlung nicht wesentlich veränderte, konnte auf eine Norminierung der Organgewichte verzichtet werden. Sowohl bei Milz (Figur 12A) als auch bei Leber (Figur 12B) konnte kein signifikanter Unterschied in den Organgewichten von LG-Acetylsalicylat behandelten Tieren zu den Kontrolltieren festgestellt werden. Somit sind nach inhalativer Behandlung von Mäusen keine Zeichen für eine Lebertoxizität, die oft mit Schwellung des Organs einhergeht zu beobachten. Weiterhin sind keine systemischen Entzündungsreaktionen, die mit einer Vergrößerung der Milz einhergehen, zu beobachten.

Aus den oben beschriebenen Studien geht zusammenfassend hervor, dass die inhalative Applikation von 2 ml LG-Acetylsalicylat mit einer Konzentration von 50 mM über einen Zeitraum von 5 Tagen für Mäuse gut verträglich ist.

### Beispiel 7: Patiententests

In einem Heilversuch wurde 4 Patienten mit bronchialen Infekten eine Lösung, enthaltend bis zu 2M LG-ASA (50:50) und zerstäubt zu Partikelgrößen von unter 5 µm, dargereicht. Die Gesamtmengen an LG-ASA betrugen bis zu 350 mg. Der Inhalationsfluss wurde auf unter 500 ml/s, meist unter 300 ml/s eingestellt. Das Atemvolumen betrug zumindest 30%, meist zumindest 50%, der Inspirationskapazität der Patienten.

Bei drei der Patienten wurde eine deutliche Verbesserung der Symptome bereits am Folgetag der Darreichung festgestellt. Bei allen Patienten war eine Verbesserung der Symptome am 3. Tag nach der Darreichung zu beobachten. Dabei war die subjektiv empfundene Verträglichkeit dieser hohen Konzentrationen in den Lösungen ausgezeichnet. Kein Patient berichtete von Geschmacksirritationen. Auch wurde kein vermehrter Hustenreiz festgestellt

## Patentansprüche

1. Zusammensetzung enthaltend ein Salz der o-Acetylsalicylsäure mit einer Aminosäure ausgewählt aus der Gruppe bestehend aus "Lysin, Arginin, Ornithin, Diaminobuttersäure, und Mischungen solcher Aminosäuren" zur Verwendung bei der Prophylaxe oder Behandlung viraler Infektionen des Menschen oder von Tieren.

2. Zusammensetzung **zur Verwendung** nach Anspruch 1, wobei zusätzlich ein Salz der o-Acetylsalicylsäure mit Glycin enthalten ist.

3. Zusammensetzung **zur Verwendung** nach Anspruch 2, wobei die basische Aminosäure D-Lysin, L-Lysin oder eine Mischung aus D-Lysin und L-Lysin ist.

4. Zusammensetzung **zur Verwendung** nach einem der Ansprüche 1 bis 3 zur Prophylaxe oder Behandlung von Infektionen mit Influenza A Viren oder Rhinoviren.

5. Zusammensetzung **zur Verwendung** nach einem der Ansprüche 1 bis 4 in galenischer Herrichtung als flüssige, wässrige Zusammensetzung, insbesondere zur aerogenen Darreichung.

6. Zusammensetzung **zur Verwendung** nach Anspruch 5, wobei die wässrige Zusammensetzung eine 0,1 bis 5 M, insbesondere 0,1 bis 3 M, vorzugsweise 1 bis 3 M, wässrige Lösung des Salzes ist.

7. Zusammensetzung **zur Verwendung** nach Anspruch 5, wobei die wässrige Zusammensetzung eine 0,01 bis 100 mM, insbesondere 0,1 bis 10 mM, wässrige Lösung des Salzes ist.

8. Zusammensetzung **zur Verwendung** nach einem der Ansprüche 1 bis 7, wobei die Zusammensetzung in einem pharmazeutisch verträglichen Behälter mit einem Zerstäuber oder Vernebler angeordnet ist.

9. Zusammensetzung **zur Verwendung** nach Anspruch 8, wobei der Behälter zur Einstellung eines Inhalationsflusses von unter 1000 ml/s, vorzugsweise unter 500 ml/s, insbesondere unter 300 ml/s, sowie eines Atemvolumens von mindestens 10%, insbesondere mindestens 30%, vorzugsweise mindestens 50%, bis zu 95%, bezogen auf die Inspirationskapazität eines Patienten, eingerichtet ist.

10. Aerosolformulierung enthaltend eine Zusammensetzung gemäß Anspruch 1 bis 5, ein Treibmittel sowie optional Hilfs- und/oder Trägerstoffe.

11. Aerosolformulierung nach Anspruch 10, wobei das Salz in einer Menge von 0,001 bis 50 Gew. -%, 0,001 bis 10 Gew. -%, insbesondere 0,1 bis 10 Gew. -%, oder insbesondere 10 bis 50 Gew. %, bezogen auf die Gesamtformulierung, enthalten ist.

12. Aerosolformulierung nach Anspruch 10 oder 11, wobei das Salz in Partikelform oder Lösung mit einem MMAD Wert von weniger als 10 µM, insbesondere weniger als 5 µM, enthalten ist.

## Claims

1. Composition containing a salt of o-acetylsalicylic acid with an amino acid selected from the group consisting of "lysine, arginine, ornithine, diaminobutyric acid and mixtures of such amino acids" for use in the prevention or treatment of viral infections in humans or animals.

2. Composition for use according to claim 1, additionally containing a salt of o-acetylsalicylic acid with glycin.

3. Composition for use according to claim 2, wherein the basic amino acid is D-lysine, L-lysine or a mixture of D-lysine and L-lysine.

4. Composition for use according to any one of claims 1 to 3 for the prevention or treatment of infections with influenza A viruses or rhino-viruses.

5. Composition for use according to any one of claims 1 to 4 in galenic preparation as liquid, aqueous composition, in particular for aerogenic application.

6. Composition for use according to claim 5, wherein the aqueous composition is a 0.1 to 5 M, in particular 0.1 to 3 M, preferably 1 to 3 M aqueous solution of the salt.

7. Composition for use according to claim 5, wherein the aqueous composition is a 0.01 to 100 mM, in particular 0.1 to 10 mM aqueous solution of the salt.

8. Composition for use according to any one of claims 1 to 7, wherein the composition is arranged in a pharmaceutically acceptable container with an atomizer or nebulizer.

9. Composition for use according to claim 8, wherein the container is designed for adjustment to an inhalation flow of less than 1,000 ml/s, preferably less than 500 ml/s, in particular less than 300 ml/s and to a respiratory volume of at least 10%, in particular at least 30%, preferably at least 50%, up to 95% in relation to the inspiratory capacity of a patient.

10. Aerosol formulation containing a composition according to claims 1 to 5, a propellant and, optionally, adjuvants and/or excipients.

11. Aerosol formulation according to claim 10, wherein the salt is contained in an amount of 0.001 to 50 % by weight, 0.001 to 10 % by weight, in particular 0,1 to 10 % by weight or in particular 10 to 50 % by weight in relation to the total formulation.

12. Aerosol formulation according to claim 10 or 11, wherein the salt is contained in form of particles or solution with an MMAD value of less than 10 µM, in particular less than 5 µM.

## Revendications

1. Composition comprenant un sel d'acide o-acétylsalicylique avec un acide aminé choisi dans le groupe constitué par « la lysine, l'arginine, l'ornithine, l'acide diaminobutyrique et des mélanges de ces acides aminés » pour l'utilisation dans la prophylaxie ou le traitement d'infections virales chez l'homme ou l'animal.

2. Composition pour l'utilisation selon la revendication 1, un sel d'acide o-acétylsalicylique avec de la glycine étant en plus présent.

3. Composition pour l'utilisation selon la revendication 2, l'acide aminé basique étant la D-lysine, la L-lysine, ou un mélange de D-lysine et de L-lysine.

4. Composition pour l'utilisation selon l'une des revendications 1 à 3 pour la prophylaxie ou le traitement des infections par les virus de la grippe A ou des rhinovirus.

5. Composition pour l'utilisation selon l'une des revendications 1 à 4 pour la réalisation galénique sous forme de composition aqueuse liquide, en particulier pour la forme galénique aérogène.

6. Composition pour l'utilisation selon la revendication 5, la composition aqueuse étant une solution aqueuse du sel de 0,1 à 5 M, en particulier de 0,1 à 3 M, de préférence de 1 à 3 M.

7. Composition pour l'utilisation selon la revendication 5, la composition aqueuse étant une solution aqueuse du sel de 0,01 à 100 mM, en particulier de 0,1 à 10 mM.

8. Composition pour l'utilisation selon l'une des revendications 1 à 7, la composition étant disposée dans un récipient pharmaceutiquement acceptable avec un pulvérisateur ou nébulisateur.

9. Composition pour l'utilisation selon la revendication 8, le récipient étant conçu pour le réglage d'un débit d'inhalation inférieur à 1000 ml/s, de préférence inférieur à 500 ml/s, en particulier inférieur à 300 ml/s ainsi qu'un volume respiratoire d'au moins 10 %, en particulier d'au moins 30 %, de préférence d'au moins 50 %, jusqu'à 95 %, par rapport à la capacité d'inspiration d'un patient.

10. Formulation en aérosol contenant la composition selon la revendication 1 à 5, un propulseur et un auxiliaire et/ou support facultatif.

11. Formulation en aérosol selon la revendication 10, le sel étant présent en une quantité allant de 0,001 à 50 % en poids, 0,001 à 10 % en poids, en particulier 0,1 à 10 % en poids, ou en particulier de 10 à 50 % en poids, par rapport à la formulation totale.

12. Formulation en aérosol selon la revendication 10 ou 11, le sel étant présent sous une forme particulaire ou sous forme de solution avec une valeur MMAD inférieure à 10 µM, en particulier inférieure à 5 µM.
